(19) Europäisches Patentamt
European Patent Office
Office européen des brevets

(11) **EP 4 241 715 A1**

(12) **EUROPEAN PATENT APPLICATION**
published in accordance with Art. 153(4) EPC

(43) Date of publication:
**13.09.2023 Bulletin 2023/37**

(21) Application number: **21888638.0**

(22) Date of filing: **04.11.2021**

(51) International Patent Classification (IPC):
**A61B 34/00** (2016.01)

(86) International application number:
**PCT/CN2021/128828**

(87) International publication number:
**WO 2022/095946 (12.05.2022 Gazette 2022/19)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **05.11.2020 CN 202011223748**

(71) Applicant: **Microport Navibot (Suzhou) Co., Ltd.
Suzhou, Jiangsu 215000 (CN)**

(72) Inventors:
• **YANG, Junjuan
Suzhou, Jiangsu 215000 (CN)**

• **GE, Yinming
Suzhou, Jiangsu 215000 (CN)**
• **HE, Chao
Suzhou, Jiangsu 215000 (CN)**
• **LI, Tao
Suzhou, Jiangsu 215000 (CN)**
• **PENG, Weili
Suzhou, Jiangsu 215000 (CN)**

(74) Representative: **Patentship
Patentanwaltsgesellschaft mbH
Elsenheimerstraße 65
80687 München (DE)**

(54) **SURGICAL ROBOT, CONTROL METHOD, SYSTEM, AND READABLE STORAGE MEDIUM**

(57)    A surgical robot, a control method, a system and a readable storage medium are disclosed. The surgical robot includes a manipulation terminal. The control method of the surgical robot includes: defining a safe zone and a warning boundary outside the safe zone, based on edge information of the surgical object; and based on a distance function between a current position of the manipulation terminal and the warning boundary, as well as on first feedback information from the manipulation terminal and second feedback information produced from an external environmental force, compensating drive information applied by the surgical robot to the manipulation terminal so that, when the manipulation terminal moves out of the safe zone, an impact of the external environmental force on driving of the manipulation terminal is reduced, eliminated or restricted.

Fig. 5

**Description**

**TECHNICAL FIELD**

The present invention relates to the field of robot-assisted surgical systems and methods and, in particular, to a surgical robot, a control method, a system, and a readable storage medium.

**BACKGROUND**

Orthopedic surgical robots can effectively reduce damage to soft and bone tissues, bleeding and trauma, and are therefore more favorable to post-operative recovery of patients' knee joints. However, in robot-assisted surgical procedures, bone cutting areas are generally determined at surgeon's discretion. Consequently, for the same patient, different surgeons may get different results and outcomes, and operational errors may occur, leading to excessive removal of soft and bone tissues.

Therefore, for an orthopedic surgical robot, it is necessary to properly determine the boundary of a bone cutting area so that the robot can be effectively limited to move within a range corresponding to the boundary. Although there are existing solutions for limiting a range of movement of a robot, such solutions requires deriving a precise dynamic model for tactile devices, which is, however, difficult to achieve for surgical robots with sophisticated mechanisms. In particular, when under the influence of friction and other nonlinear factors, it is probable for surgeons to make incorrect determinations.

**SUMMARY OF THE INVENTION**

It is an object of the present invention to provide a surgical robot, a control method, a system, and a readable storage medium, which overcome the problem of difficult, inaccurate conventional boundary control for surgical robot, which tends to lead to operational errors.

The above object is attained by a control method for a surgical robot comprising a manipulation terminal proposed in a first aspect of the present invention, which comprises:

defining a safe zone and an warning boundary outside the safe zone, based on edge information of a surgical object; and

based on a distance function between a current position and posture of the manipulation terminal and the warning boundary, as well as on first feedback information from the manipulation terminal and second feedback information produced from an external environmental force, compensating drive information applied by the surgical robot to the manipulation terminal so that, when the manipulation terminal moves out of the safe zone, an impact of the external environmental force on driving of the manipulation terminal is reduced, eliminated or restricted.

Optionally, the first feedback information may comprise commanded position and posture information for a joint in the manipulation terminal, and the second feedback information may comprise torque information of the external environmental force on the joint in the manipulation terminal.

Optionally, compensating the drive information applied by the surgical robot to the manipulation terminal may comprise:

deriving a commanded angle $\theta$ for the joints in the manipulation terminal from the commanded position and posture information Xd through inverse kinematics;

calculating a theoretical output torque Fs by using the commanded angle $\theta$ as an input to a dynamic calculation;

calculating a torque required by the joint in the manipulation terminal for movement from a current position and posture to the commanded position and posture by using the commanded angle $\theta$ as an input to a position and posture controller;

calculating a torque Fc of the external environmental force from an equivalent torque F sensed by a force sensor under an action of the external environmental force, gravity and friction compensation torques N and the theoretical output torque Fs; and

obtaining the drive information through compensating the torque required by the joint in the manipulation terminal for movement from the current position and posture to the commanded position and posture with the torque Fc of the external environmental force.

Optionally, the external environmental force may comprise a resistance torque Fa of a surgical object to the manipulation terminal and a traction torque f applied by an operator to the manipulation terminal, wherein the equivalent torque F satisfy F= Fs+N+Fa+f and the torque Fc of the external environmental force satisfy Fc= F-Fs-N.

Optionally, the calculation performed by the position and posture controller may comprise:
calculating the torque required by the joint in the manipulation terminal for movement from the current position and posture to the commanded position and posture based on commanded and current positions and postures and commanded and current speeds of the joint in the manipulation terminal.

Optionally, the commanded speed may be obtained by performing a differential calculation on the commanded position and posture.

Optionally, the first feedback information may comprise commanded position and posture information for the joint in the manipulation terminal, wherein the first feedback information comprises an impedance control model of the external environmental force over the joint in the manipulation terminal.

Optionally, compensating the drive information applied by the surgical robot to the manipulation terminal may comprise:

> deriving a commanded angle $\theta$ for the joint in the manipulation terminal from commanded position and posture information Xd through inverse kinematics;
> calculating theoretical output torque Fs by using the commanded angle $\theta$ as an input to a dynamic calculation;
> calculating a first torque in Cartesian space using the impedance control model based on a position and posture difference between current and commanded positions and postures of the manipulation terminal and a speed difference between current and commanded speeds of the manipulation terminal;
> converting the first torque into a second torque that the individual joint is subject to through a transposition of a Jacobian matrix of the joint at a current angle thereof;
> deriving a third torque of the individual joint through compensating the individual joint in the manipulation terminal with a corresponding friction feedforward f; and
> deriving the drive information from the theoretical output torque Fs, the third torque and the second torque.

Optionally, an input to the impedance control model may be derived using a process comprising the steps of:

> calculating a position and posture variation for the joint through admittance control from an equivalent torque F output from a force sensor under an action of the external environmental force;
> calculating the position and posture difference between the current and commanded positions and postures of the manipulation terminal from the position and posture variation through forward kinematics; and
> taking the position and posture difference as the input to the impedance control model.

Optionally, the manipulation terminal may comprise a robotic arm and/or a manipulator, wherein the first feedback information comprises commanded position and posture information for a joint in the robotic arm and/or the manipulator, and wherein the manipulator is configured to fix a surgical instrument thereto and guide it to perform a surgical operation.

The above object is also attained by a readable storage medium proposed in a second aspect of the present invention, which stores a program thereon. When executed, the program implements a control method for a surgical robot as defined above.

The above object is also attained by a surgical robot proposition and postured in a third aspect of the present invention, which comprises a manipulation terminal. The manipulation terminal comprises a robotic arm and/or a manipulator for guiding a surgical instrument to perform a surgical operation. The manipulation terminal is controlled using a control method for a surgical robot as defined above.

The above object is also attained by a surgical robot system proposed in a fourth aspect of the present invention, which comprises a control device, a navigation device and a manipulation terminal. The navigation device is configured to track a current position and posture of the manipulation terminal and feed the position and posture information back to the control device. The control device is configured to control the manipulation terminal using a control method for a surgical robot as defined above.

Optionally, the manipulation terminal may comprise a robotic arm and a manipulator for guiding a surgical instrument to perform a surgical operation, the manipulator having a plurality of degrees of freedom, wherein the first feedback information comprises commanded position and posture information for a joint in the robotic arm and/or the manipulator.

In summary, the present invention provides a surgical robot, a control method for the surgical robot, a surgical robot system, and a readable storage medium. The surgical robot includes a manipulation terminal, and the control method for the surgical robot includes: defining a safe zone and a warning boundary outside the safe zone, based on edge information of the surgical object; and based on a distance function between a current position of the manipulation terminal and the warning boundary, as well as on first feedback information from the manipulation terminal and second feedback information produced from an external environmental force, compensating drive information applied by the surgical robot to the manipulation terminal so that, when the manipulation terminal moves out of the safe zone, an impact of the external environmental force on driving of the manipulation terminal is reduced, eliminated or restricted.

With this arrangement, through compensating the drive information applied to the manipulation terminal with the first feedback information and the second feedback information, an actuating joint is reversely compensated for the external environmental force. In this way, such boundary control is achieved that an impact of the external environmental force on a patient is minimized and after the manipulation terminal moves out of the safe zone, an additional torque required to drive a joint in a robotic arm, as well as the external environmental force, must be increased to obtain the same effect, thereby avoiding possible operational errors of the operator.

**BRIEF DESCRIPTION OF THE DRAWINGS**

Those of ordinary skill in the art would appreciate that the accompanying drawings are provided to facilitate a better understanding of the present invention and do not limit the scope thereof in any sense, in which:

Fig. 1 schematically illustrates a surgical scenario in which the present invention is applicable;
Fig. 2 schematically illustrates degrees of freedom of an osteotomy guide according to Embodiment 1 of the present invention;
Fig. 3 schematically illustrates the osteotomy guide according to Embodiment 1 of the present invention;
Fig. 4 schematically illustrates a surgical robot according to Embodiment 1 of the present invention;
Fig. 5 is a schematic block diagram of a control method according to Embodiment 1 of the present invention;
Fig. 6 is a schematic block diagram of a control method according to Embodiment 2 of the present invention;
Fig. 7 schematically illustrates a physical impedance control model according to Embodiment 2 of the present invention;
Fig. 8 is a schematic diagram of impedance control according to Embodiment 2 of the present invention; and
Fig. 9 is a schematic diagram of admittance control according to Embodiment 2 of the present invention.

In these figures,
1 denotes a surgical cart; 2, a robotic arm; 3, a guide fiducial; 4, an osteotomy guide; 5, an osteotomy tool; 6, a tracker; 7, a secondary monitor; 8, a primary monitor; 9, a navigation cart; 10, a keyboard; 11, a femoral fiducial; 12, a femur; 13, a tibial fiducial; 14, a tibia; 15, a base fiducial; 16, an X translational axis; 17, a Y translational axis; and 18, a Z axis.

## DETAILED DESCRIPTION

Objects, advantages and features of the present invention will become more apparent upon reading the following more detailed description of the present invention, which is set forth by way of particular embodiments with reference to the accompanying drawings. Note that the figures are provided in a very simplified form not necessarily drawn to exact scale and for the only purpose of facilitating easy and clear description of the embodiments. In addition, the structures shown in the figures are usually partial representations of their actual counterparts. In particular, as the figures would have different emphases, they are sometimes drawn to different scales.

As used herein, the singular forms "a", "an" and "the" include plural referents, and the term "or" is generally employed in the sense of "and/or", "several" of "at least one", and "at least two" of "two or more than two". Additionally, the use of the terms "first", "second" and "third" herein is intended for illustration only and is not to be construed as denoting or implying relative importance or as implicitly indicating the numerical number of the referenced item. Accordingly, defining an item with "first", "second" or "third" is an explicit or implicit indication of the presence of one or at least two of the items. As used herein, the term "proximal" generally refer to an end closer to an operator, and the term "distal" generally refer to an end closer to a subject being operated on. The terms "one end" and "the other end", as well as "proximal end" and "distal end", are generally used to refer to opposing end portions including the opposing endpoints, rather than only to the endpoints, unless the context clearly dictates otherwise. Those of ordinary skill in the art can understand the specific meanings of the above-mentioned terms herein, depending on their context.

Essentially, the present invention seeks to provide a surgical robot, a control method, a system, and a readable storage medium, which overcome the problem of difficult, inaccurate conventional boundary control for surgical robot, which tends to lead to operational errors. A detailed description is set forth below with reference to the accompanying drawings.

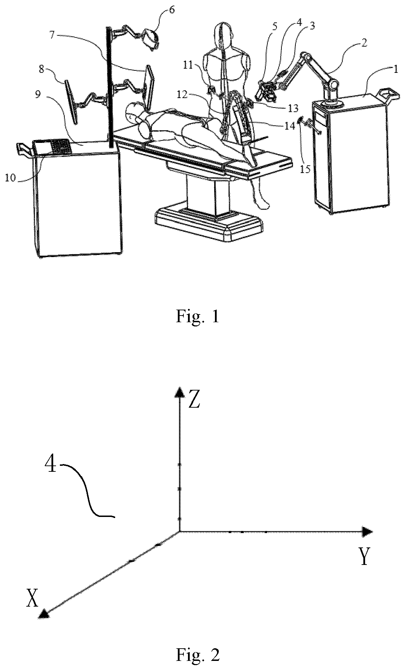

Fig. 1 shows an exemplary embodiment, in which a surgical robot embodying the present invention is used in a knee replacement scenario. However, the surgical robot is not limited to being used in any particular environment, as it can also be used in other types of surgery, such as limb surgery, abdomen surgery, chest surgery, brain surgery, etc. In the following, the surgical robot will be described in the context of use for knee replacement as an example. However, this shall not be construed as limiting the present invention in any sense.

As shown in Fig. 1, the surgical robot system includes a control device, a navigation device, a robotic arm 2 and an osteotomy guide 4. The robotic arm 2 is placed on a surgical cart 1. The control device is implemented as a computer in some embodiments, but the present invention is not so limited. The computer is equipped with a processor, a primary monitor 8 and a keyboard 10. More preferably, it further includes a secondary monitor 7. The secondary monitor 7 may display the same content as the primary monitor 8, or not. The navigation device may be a navigator based on magnetic positioning, a navigator or sensor based on optical positioning, or a navigator based on inertial positioning. Preferably, the navigation device is a navigator based on optical positioning, which provides higher measurement accuracy and enables the osteotomy guide 4 to have increased positioning accuracy, compared to other navigation techniques. The following description is set forth in the context of a navigator based on optical positioning, but this should not be construed as limiting in any way.

Specially, the navigation device includes navigation markers and a tracker 6. The navigation markers include a base fiducial 15 and a guide fiducial 3. The base fiducial 15 is kept stationary. For example, the base fiducial 15 may be fixed to the surgical cart 1 in order to provide a base coordinate system (or base fiducial coordinate system). The guide fiducial 3 is mounted on the osteotomy guide 4 to enable positional tracking of the osteotomy guide 4. The osteotomy guide 4 is mounted at an end of the robotic arm 2 so that the robotic arm 2 supports the osteotomy guide 4 and can adjust the position and orientation of the osteotomy guide 4 in space.

In practice, the tracker 6 is used to capture a signal reflected from the guide fiducial 3 (which is preferred to be a reflection of an optical signal from the tracker 6) and record a position and posture of the guide fiducial 3 (, i.e., its position and orientation in the base coordinate system). A computer program stored in a memory of the control device then control, based on the current and desired positions and postures of the guide fiducial 3, movement of the robotic arm 2. As a result, the robotic arm 2 drives the osteotomy guide 4 and the guide fiducial 3 to move until the guide fiducial 3 reaches the desired position and posture, which are mapped to a desired position and posture of the osteotomy guide 4.

Thus, in applications of the surgical robot, the osteotomy guide 4 can be automatically positioned. Moreover, during surgery, the guide fiducial 3 tracks and feeds back in real time the position and posture of the osteotomy guide 4, based on which, the robotic arm 2 is controlled to move to make positional and postural adjustments to the osteotomy guide 4 and hence to a surgical instrument mounted on the osteotomy guide 4 (e.g., a swing saw or an electric drill). In this way, in addition to high positioning accuracy of the osteotomy guide 4 being achievable, the osteotomy guide 4 is supported on the robotic arm 2 rather than fixed on a patient's body, thereby avoiding causing secondary damage thereto.

Generally, the surgical robot further includes the surgical cart 1 and a navigation cart 9. The control device and part of the navigation device are mounted on the navigation cart 9. For example, the processor may be deployed inside the navigation cart 9, while the keyboard 10 may be arranged outside the navigation cart 9 to facilitate manipulation. Additionally, the primary monitor 8, the secondary monitor 7 and the tracker 6 may be all mounted on a mast erected upright on a surface of the navigation cart 9, with the robotic arm 2 being mounted on the surgical cart 1. The use of the surgical cart 1 and the navigation cart 9 enables easy operation throughout a surgical procedure.

The use of the surgical robot in this embodiment for knee replacement surgery generally involves the steps as follows.

Step SK1: Movement of the surgical cart 1 and the navigation cart 9 to respective proper locations beside a hospital bed.

Step SK2: Deployment of the navigation markers (further including a femoral fiducial 11 and a tibial fiducial 13), the osteotomy guide 4 and other necessary components (e.g., a sterile bag).

Step SK3: Preoperative planning. Specifically, an operator may achieve the preoperative planning by importing a CT/MRI scan model of a patient's bone to the computer, which then develops an osteotomy plan including, for example, coordinates of a bone surface to be cut, a model of a prosthesis, a target position and posture for the prosthesis and other information. Specifically, based on image data of the patient's knee joint obtained from a CT/MR scan, a virtual three-dimensional (3D) model of the knee joint may be created, and the osteotomy plan may be developed based on the virtual 3D knee joint model and serve as a basis for the operator to carry out preoperative assessment. More specifically, the osteotomy plan may be developed based on the virtual 3D knee joint model, as well as on dimensions of the prosthesis, a target location for an osteotomy plate and the like, and may be finally output in the form of a surgical report, which may specify a series of reference data including the coordinates of the bone surface to be cut, an amount of bone to be cut away, an osteotomy angle, the dimensions of the prosthesis, the target location for the prosthesis, auxiliary surgical instruments, etc. In particular, it may contain a series of passages of descriptive text for the surgical operator's reference, which may specify the reason(s) for the osteotomy angle, for example. The virtual 3D knee joint model may be displayed on the primary monitor 8. During the preoperative planning, the operator may input surgical parameters through the keyboard 10.

Step SK4: Real-time bone registration. In this embodiment, the navigation markers further includes a femoral fiducial 11 and a tibial fiducial 13. The femoral fiducial 11 is used to determine the position and posture of a femur 12 in space. Likewise, the tibial fiducial 13 is used to determine the position and posture of a tibia 14 in space. Subsequent to the preoperative assessment, positions of feature points of the patient's femur 12 and tibia 14 may be acquired in real time, and the processor may then determine actual positions and postures of the bones using a feature matching algorithm and then correlate them to their respective graphic representations. After that, the navigation device may associate the actual positions and postures of the femur 12 and tibia 14 with the corresponding fiducial markers mounted thereon, thereby enabling the femoral fiducial 11 and the tibial fiducial 13 to track the actual positions of the bones in real time. Through associating the actual positions and postures of the femur 12 and tibia 14 with the corresponding fiducial markers mounted thereon by the navigation device, the femoral fiducial 11 and the tibial fiducial 13 can track the actual positions of the bones in real time, and during surgery, as long as the fiducial marks remain stationary relative to the respective bones on which they are arranged, displacements of the bones will not affect the surgical outcomes.

Step SK5: Deployment of the robotic arm 2 in position for surgical operation. Specifically, the navigation device sends the coordinates of the bone surface to be cut that are determined in the preoperative planning to the robotic arm 2, which then locates the bone surface to be cut with the aid of the guide fiducial 3 and moves to a proper location. After causing the robotic arm 2 to remain stationary (i.e., in an immobilized state), the operator may perform bone cutting and/or drilling operations using an osteotomy tool 5 such as a swing saw or an electric drill, with the aid of the osteotomy guide 4 for guidance, securing or locating. Following the completion of the bone cutting and/or drilling operations, the operator may set the prosthesis in place and carry out other necessary operations.

Traditional surgery systems and navigated surgery systems without the participation of a robotic arm in positioning require manual adjustment and positioning of an osteotomy guide, which is, however, inaccurate and inefficient. In contrast, by positioning the osteotomy guide 4 with the robotic arm 2, the operator needs not to fix the osteotomy guide on a bone with additional bone screws, reducing trauma to the patient and surgical time. As noted above, the guide fiducial 3 may be mounted on the osteotomy guide 4, but in other embodiments, the guide fiducial 3 may also be mounted on a terminal joint of the robotic arm 2.

Robot-assisted surgery can be achieved based on the above-discussed surgical robot, which can facilitate an osteotomy procedure by helping an operator identify a target site in need of osteotomy, or identify an osteotomy tool. However, during an osteotomy procedure, for example, once immobilization of the robotic arm 2 is achieved, it is difficult to additionally limit the positions and posture of the osteotomy guide 4 any longer to prevent them from being influenced by an external environmental force. Therefore, it is hard to effectively limit movement of the osteotomy guide 4 within a defined range, and operational errors that might cause unnecessary damage to the patient may occur.

On the basis of this, embodiments of the present invention provide a control method for a surgical robot incorporating a manipulation terminal. It would be appreciated that the manipulation terminal includes at least one of the robotic arm 2 and a manipulator (for guiding a surgical instrument to perform a surgical procedure, such as the osteotomy guide 4), or a combination of both. The method is used to control movement of the manipulation terminal. In other application scenarios, the manipulator is not limited to the osteotomy guide 4, and can be alternatively implemented as other devices capable of limiting the range of movement of the manipulation terminal of the surgical robot. The surgical robot is controlled by the method.

EMBODIMENT 1

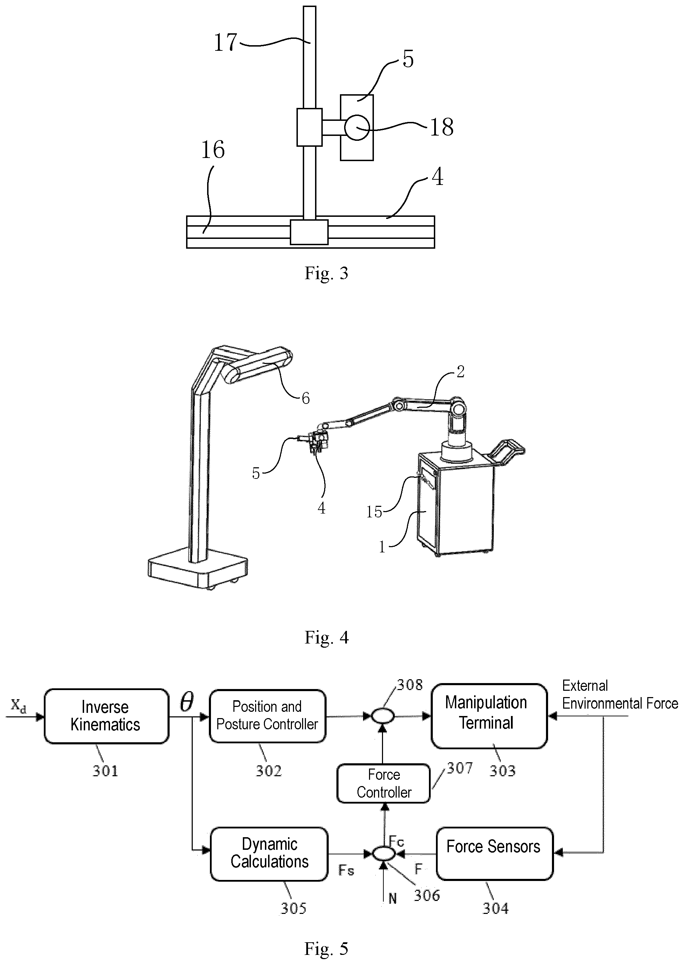

Reference is now made to Figs. 2 to 5. Fig. 2 schematically illustrates degrees of freedom of an osteotomy guide according to Embodiment 1 of the present invention. Fig. 3 schematically illustrates the osteotomy guide according to Embodiment 1 of the present invention. Fig. 4 schematically illustrates a surgical robot according to Embodiment 1 of the present invention. Fig. 5 is a block diagram showing principles of a control method according to Embodiment 1 of the present invention.

In Embodiment 1, the osteotomy guide 4 is implemented as a manipulation terminal, as an example. In practice, the osteotomy guide and/or joints of a robotic arm may also be controlled. Figs. 2 to 3 show the osteotomy guide 4, which has three degrees of freedom, namely, a translational degree of freedom along an X axis, a translational degree of freedom along a Y axis and a rotational degree of freedom about a Z axis in Fig. 2. Fig. 3 is a schematic top view of the osteotomy guide 4. As shown, for the osteotomy guide 4, there are defined the X translational axis 16, the Y translational axis 17 and the Z axis 18 that is perpendicular to the X translational axis 16 and the Y translational axis 17. After an osteotomy tool 5 (e.g., a swing saw) is mounted on the osteotomy guide 4, it can translate along the X translational axis 16 and the Y translational axis 17 and rotate about the Z axis 18. The X translational axis 16, the Y translational axis 17 and Z axis 18 can be considered as corresponding to three joints of the osteotomy guide 4. Preferably, all the three joints can acquire drive information from a control device and perform actions based on the drive information. For example, the osteotomy guide 4 may include three joint driving motors that enable the three degrees of freedom. In some other embodiments, the manipulation terminal may be alternatively implemented as a robotic arm 2 also including several joint driving motors. Of course, in some embodiments, the manipulation terminal may include both the robotic arm 2 and the osteotomy guide 4.

Further, referring to Fig. 4, a tracker 6 can identify, through a base fiducial 15, the current spatial position and posture of the osteotomy tool 5. Specifically, assuming that the robotic arm 2 is kept stationary (i.e., immobilized) at a certain location, let $^{robot}_{version}A$ denote a position and posture of the robotic arm as identified by the tracker 6 (which is derived by matrix transformation from position and posture information that is obtained by the control device from a joint encoder in the robotic arm), and let $^{tool}_{version}B$ denote a position and posture of the osteotomy guide 4 as identified by the tracker 6 (which is computationally derived from data acquired by the tracker 6 during its tracking of a guide fiducial 3 on the osteotomy guide 4), the position and posture $^{tool}_{robot}T$ of the osteotomy tool 5 depending simply on movement of the osteotomy guide 4 can be expressed as:

$$^{tool}_{robot}T = {}^{robot}_{version}A^{-1} * {}^{tool}_{version}B$$

Thus, it would be appreciated that the tracker 6 is able to track the position and posture depending simply on movement of the osteotomy guide 4. As the osteotomy tool 5 is mounted on the osteotomy guide 4, the position and posture of the osteotomy guide 4 just reflect those of the osteotomy tool 5.

A control method for the surgical robot includes the steps as follows.

Step S 1: Defining a safe zone and a warning boundary encircling the safe zone, based on edge information of a surgical object.

Step S2: Based on a distance function between the current position and posture of a surgical instrument (e.g., the osteotomy tool 5) mounted on the manipulation terminal (e.g., the osteotomy guide 4) and the warning boundary, as well as on first feedback information from the manipulation terminal and second feedback information produced from an external environmental force, compensate drive information applied by the surgical robot to the manipulation terminal so that, when the manipulation terminal move out of the safe zone, the influence of the external environmental force on the driving of the manipulation terminal is reduced, eliminated or restricted.

In an exemplary implementation, the surgical object may be a bone, for example. In step S 1, a safe zone and a warning boundary are defined. For example, in some implementations, edge information of the bone may be obtained by an image acquisition device (e.g., a scanning device such as a CT scanner), and the safe zone and the warning boundary may be defined based on the edge information of the bone. Specifically, the image acquisition device may capture an environmental boundary of the bone. An operator (e.g., a surgeon) may formulate a preoperative plan based on his/her own experience, and define the warning boundary and the safe zone based on the environmental boundary (in such a manner that the safe zone is encompassed by the environmental boundary, which is in turn encompassed by the warning boundary, wherein the innermost safe zone is area ensuring safe surgical operation).

In step S2, the first feedback information contains commanded position and posture information for joints of the robotic arm 2 and/or the osteotomy guide 4, and the second feedback information contains torque information on the osteotomy guide 4 calculated from the external environmental force acting on the joints of the robotic arm 2 and/or the osteotomy guide 4.

In this embodiment, based on a distance function between the current position of the osteotomy guide 4 (which can be obtained by tracking the position and posture of the osteotomy guide 4 by the tracker 6) and the warning boundary, as well as on position and posture information of the joints of the robotic arm 2 and/or the osteotomy guide 4 and the torque information on the osteotomy guide 4 derived from the external environmental force, a torque compensation control mode is employed to compensate drive information applied by the control device to the osteotomy guide 4, thereby minimizing or eliminating the influence of the external environmental force on the bone. Optionally, examples of the warning boundary may include a warning line, a warning surface and the like. Those skilled in the art may establish the distance function between the current position of the osteotomy guide 4 and the warning boundary. In particular implementations, the distance function may be established by causing the osteotomy guide 4 to move toward the safe zone and stopping it when it reaches the safe zone. As noted above, the osteotomy guide 4 has three joints that enable three degrees of freedom. That is, three degrees of freedom are further provided, in addition to those of the joints in the robotic arm 2. For example, if the robotic arm 2 has 6 degrees of freedom, then the robotic arm 2 and the osteotomy guide 4 will have a total of 9 degrees of freedom. This can result in a significant increase in surgical flexibility.

Optionally, the external environmental force may include resistance from the surgical object (e.g., a bone) to the manipulation terminal (e.g., the osteotomy guide 4) and traction exerted by the operator on the osteotomy guide 4. Specifically, the traction exerted by the operator on the osteotomy guide 4 may be a pushing force, a pulling force or the like applied by his/her hand.

The external environmental force may be measured, for example, by the force sensor 304 and output in the form of an equivalent torque F. Examples of the force sensor 304 may include, but are not limited to, six-dimensional force sensor, joint torque sensor and the like, and the force sensor 304 may be arranged on the osteotomy guide 4. The force sensor 304 can measure traction exerted by the operator on the osteotomy guide 4 and resistance from the bone to the osteotomy guide 4 transmitted through the osteotomy tool 5. Of course, the external environmental force may also be derived as the equivalent torque F from electrical current through the joint driving motor for the osteotomy guide 4.

Referring to Fig. 5, the compensation of the drive information applied by the surgical robot to the osteotomy guide 4 may include the steps as follows.

Step SA1: Deriving commanded angles θ for the joints of the osteotomy guide 4 from the commanded position and posture information Xd through inverse kinematics 301. The commanded position and posture refers to a target position and posture sent from a control system of the surgical robot to the osteotomy guide 4, and the commanded angles θ refer to target angles sent from the control system of the surgical robot to the joints of the osteotomy guide 4.

Step SA2: Deriving theoretical output torques Fs through dynamic calculations 305 with the commanded angles θ as inputs. Specifically, the commanded angles θ may be decomposed into commanded positions and commanded speeds for the joints of the osteotomy guide 4, from which the theoretical output torques Fs for the joints of the osteotomy guide 4 can be derived through the dynamic calculations 305.

Step SA3: With the commanded angles θ as inputs to a position and posture controller 302, calculating torques required by the joints of the osteotomy guide 4 for movement from the current position and posture to the commanded position and posture (i.e., the target position and posture). This calculation performed by the position and posture controller 302 may include: calculating the torques required by the joints of the osteotomy guide 4 for movement from the current position and posture to the commanded position and posture based on the commanded and current positions and postures and the commanded and current speeds of the joints of the osteotomy guide 4. Preferably, the commanded speeds may be calculated by performing differential calculations on the commanded positions and postures.

Step SA4: Calculating a torque Fc of the external environmental force based on the equivalent torque F output from the force sensor 304 under the action of the external environmental force, a gravity and friction compensation torque N and the theoretical output torque Fs (see 306 in Fig. 5 for reference). Here, the torque Fc of the external environmental force may be taken as a resultant torque of a traction torque f applied by the operator to the osteotomy guide 4 and a resistance torque Fa applied by the bone to the osteotomy guide 4 through the osteotomy tool 5. Optionally, the equivalent torque F may satisfy F= Fs+N+Fa+f, and the torque Fc of the external environmental force may satisfy Fc= F-Fs-N. Further, the calculated theoretical torque Fc of the external environmental force may be processed in a force controller 307 so as to be closer to the actual values.

Step SA5: Compensating for the torques required by the joints of the osteotomy guide 4 for movement from the current position and posture to the commanded position and posture with the torque Fc of the external environmental force (see 308 in Fig. 5 for reference), thereby obtaining the drive information for control of the manipulation terminal (see 303 in Fig. 5 for reference). Specifically, desired torque compensations for the joints of the osteotomy guide 4 may be calculated according to the distance function and applied to the individual joints, thereby obtaining drive information for the joints. Upon the osteotomy guide 4 reaching the warning boundary, the system will automatically increase resistance of the joints to the operator's traction, thereby minimizing an impact of the traction on, and providing protection to, the bone surface being cut. Optionally, during surgery, the osteotomy guide 4 may operate in the safe zone in normal conditions and be restricted in speed upon crossing a boundary of the osteotomy guide 4. Moreover, upon reaching the warning boundary, the system controls the osteotomy guide 4 according to the distance function so that it moves along, or stops at, the warning boundary. In this way, the influence of the external environmental force on the driving of the osteotomy guide 4 after the osteotomy guide 4 moves out of the safe zone can be reduced.

In this embodiment, there is also provided a readable storage medium storing a program thereon, which, when executed, implements the control method as defined above. The readable storage medium may be integrated in the surgical robot, for example, in the control device. Alternatively, it may be attached as a separate component.

In this embodiment, there is further provided a surgical robot system comprising a control device, a navigation device and a manipulation terminal. The navigation device is configured to track the current position and posture of the manipulation terminal and feed information about the position and posture back to the control device. The control device is configured to control the manipulation terminal according to the method as defined above. Preferably, in the surgical robot system, the manipulation terminal includes a robotic arm and a manipulator for guiding a surgical instrument to perform a surgical operation. The manipulator has multiple degrees of freedom, and the first feedback information includes commanded position and posture information of joints in the robotic arm and/or the manipulator.

In summary, through compensating the drive information applied to the manipulation terminal with the first feedback information and the second feedback information, the actuating joints are reversely compensated for the external environmental force. In this way, such boundary control is achieved that an impact of the external environmental force on a patient is minimized and after the manipulation terminal moves out of the safe zone, the external environmental force must be increased to obtain the same effect, avoiding possible operational errors of the operator.

EMBODIMENT 2

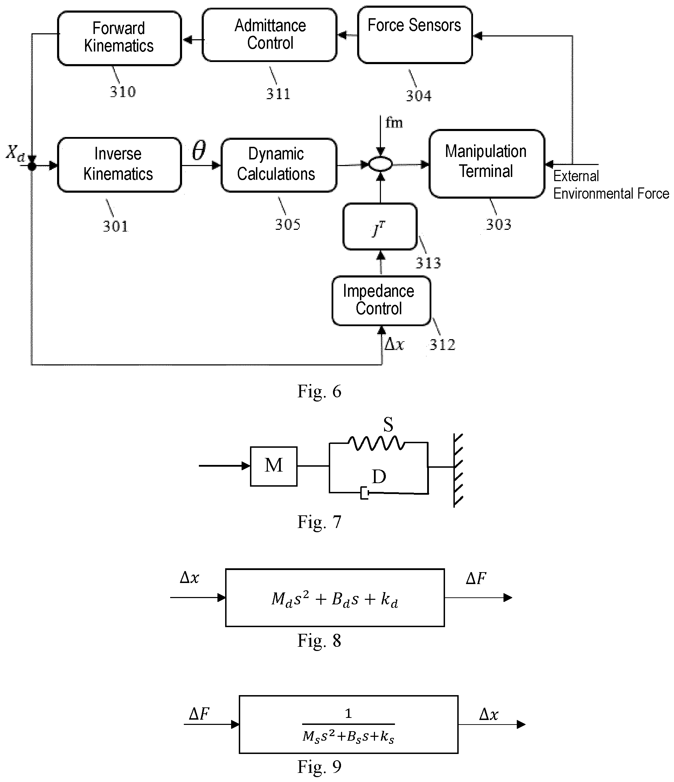

Reference is now made to Figs. 6 to 9. Fig. 6 is a block diagram showing principles of a control method according to Embodiment 2 of the present invention.

Fig. 7 schematically illustrates a physical impedance control model according to Embodiment 2 of the present invention. Fig. 8 is a schematic diagram of impedance control according to Embodiment 2 of the present invention. Fig. 9 is a schematic diagram of admittance control according to Embodiment 2 of the present invention.

Embodiment 2 of the present invention provides a surgical robot, a control method, a system, and a readable storage medium, which are substantially the same as the surgical robot, the control method, the system, and the readable storage medium provided in Embodiment 1, respectively. Below, only different features will be described, and description of those common to the two embodiments will be omitted.

In the control method provided in Embodiment 2, essentially, an impedance control mode is employed to compensate for traction applied by an operator. Specifically, in step S2, the first feedback information includes commanded position and posture information for the joints in the manipulation terminal, and the second feedback information includes an impedance control model of the external environmental force over the joints of the manipulation terminal.

Similarly, in Embodiment 2, the surgical object is implemented as a bone and the osteotomy guide 4 as the manipulation terminal, as an example.

Referring to Fig. 6, the compensation of the drive information applied by the surgical robot to the osteotomy guide 4 may include the steps as follows.

Step SB1: Deriving commanded angles θ for the joints of the osteotomy guide 4 from the commanded position and posture information Xd through inverse kinematics 301.

Step SB2: Deriving theoretical output torques Fs through dynamic calculations 305 with the commanded angles θ as inputs. Reference can be made to the above description of steps SA1 and SA2 in Embodiment 1 for more details of the meanings of the commanded angles θ and the theoretical output torques Fs and how they are obtained.

Step SB3: Based on a position and posture difference between the current and commanded positions and postures of the osteotomy guide 4 and a speed difference between the current and commanded speeds of the osteotomy guide 4, calculating first torques in Cartesian space according to the impedance control model 312. The first torques can be taken as calculated virtual torques in Cartesian space.

Step SB4: Converting the first torques into second torques of the joints in the osteotomy guide 4 according the transposition $j^T$ of a Jacobian matrix of the current angles of the joints (see 313 in Fig. 6 for reference). Specifically, the second torques may be taken as torque compensations for the joints obtained by conversion through multiplying the first torques by the transposition of the Jacobian matrix.

Step SB5: Compensating the joints of the osteotomy guide 4 with corresponding friction feedforwards fm, obtaining third torques for the joints. Specifically, the friction feedforwards fm may be calculated from speed information fed back from the joints in the osteotomy guide 4. Feedforward torque compensation can be effectuated through applying the friction feedforwards fm to the joints of the osteotomy guide 4.

Step SB6: From the theoretical output torques Fs, the third torques and the second torques, deriving the drive information for control of the osteotomy guide 4 (see 303 in Fig. 6 for reference). Specifically, the joints of the osteotomy guide 4 are subject to resultant torques of: 1) the theoretical output torques Fs; 2) the second torques derived from the first torques; and 3) friction compensations for the joints (i.e., the third torques).

Preferably, Fig. 7 shows a physical impedance control model, and Fig. 8 shows a corresponding schematic diagram. In Fig. 7, M represents the mass of the physical model; the wavy line S on the right thereof represents a spring; and D represents damping. Fig. 8 shows the expression of a transfer function in control theory, in which Md is a mass parameter corresponding to the mass of the physical model in Fig. 7, Bd is a damping coefficient corresponding to the damping of the physical model in Fig. 7, and Kd is a coefficient of resilience corresponding to the spring of the physical model in Fig. 7. Those skilled in the art can understand the impedance control model based on the knowledge in the art, and further detailed description thereof is omitted herein.

An input to the impedance control model is derived using a method including the steps as follows:
Step SC1: From the equivalent torques F output from the force sensors 304 under the action of the external environmental force, calculating position and posture variations corresponding to the joints through admittance control 311. Fig. 9 is a schematic diagram of admittance control 311, also showing the expression of a transfer function in control theory, in which Ms is a mass parameter corresponding to the mass of the physical model in Fig. 7, Bs is a damping coefficient corresponding to the damping of the physical model in Fig. 7, and Ks is a coefficient of resilience corresponding to the spring of the physical model in Fig. 7. For more details of the meaning and derivation of the equivalent torques F, reference can be made to the above description of Embodiment 1.

Step SC2: Based on the position and posture variations, calculating a position and posture difference between the actual and commanded positions and postures of the osteotomy guide 4 through forward kinematics 310.

Step SC3: Taking the position and posture difference as the input to the impedance control model. In this way, the closer the osteotomy guide 4 is to the warning boundary, the greater additional torques are required to actuate the joints, and the greater an external force is required to be applied by the operator, thereby reducing the risk of operational errors during a surgical procedure.

In the foregoing embodiments, the manipulation terminal is implemented as the osteotomy guide 4. Since the osteotomy guide 4 has only three degrees of freedom, this is conducive to the establishment of simpler kinematic and dynamic models and hence to the implementation of inverse kinematics 301, forward kinematics 310 and dynamic calculations 305. However, in some other embodiments, the manipulation terminal may be alternatively implemented as the robotic arm 2, or as a combination of the robotic arm 2 and the osteotomy guide 4. It would be appreciated that in case of more than three degrees of freedom, in addition to torques, corresponding forces must also be taken into account in the above methods of control. It would be also appreciated that, in some other embodiments, the methods of control are not limited to knee replacement surgery applications.

The embodiments disclosed herein are described in a progressive manner, with the description of each embodiment focusing on its differences from others. Reference can be made between the embodiments for their identical or similar features. Additionally, features of different embodiments may be combined with one another, without limiting the scope of the present invention.

In summary, the present invention provides a surgical robot, a control method for the surgical robot, a surgical robot system, and a readable storage medium.

The surgical robot includes a manipulation terminal, and the control method for the surgical robot includes: defining a safe zone and a warning boundary encircling the safe zone, based on edge information of the surgical object; and based on a distance function between a current position and posture of the manipulation terminal and the warning boundary, as well as on first feedback information from the manipulation terminal and second feedback information produced from an external environmental force, compensating drive information applied by the surgical robot to the manipulation terminal so that, when the manipulation terminal moves out of the safe zone, an impact of the external environmental force on driving of the manipulation terminal is reduced, eliminated or restricted. With this arrangement, through compensating the drive information applied to the manipulation terminal with the first feedback information and the second feedback information, an actuating joint is reversely compensated for the external environmental force. In this way, such boundary control is achieved that an impact of the external environmental force on a patient is minimized and after the manipulation terminal moves out of the safe zone, an additional torque required to drive a joint in a robotic arm, as well as the external environmental force, must be increased to obtain the same effect, thereby avoiding possible operational errors of the operator.

The description presented above is merely that of a few preferred embodiments of the present invention and does not limit the scope thereof in any sense. Any and all changes and modifications made by those of ordinary skill in the art based on the above teachings fall within the scope as defined in the appended claims.

**Claims**

1.  A control method for a surgical robot, the surgical robot comprising a manipulation terminal, wherein the control method comprising:

    defining a safe zone and a warning boundary outside the safe zone, based on edge information of a surgical object; and
    based on a distance function between a current position and posture of the manipulation terminal and the warning boundary, as well as on first feedback information from the manipulation terminal and second feedback information produced from an external environmental force, compensating drive information applied by the surgical robot to the manipulation terminal so that, when the manipulation terminal moves out of the safe zone, an impact of the external environmental force on driving of the manipulation terminal is reduced, eliminated or restricted.

2.  The control method for the surgical robot of claim 1, wherein the first feedback information comprises commanded position and posture information for a joint in the manipulation terminal and in that the second feedback information comprises torque information of the external environmental force on the joint in the manipulation terminal.

3.  The control method for the surgical robot of claim 2, wherein compensating the drive information applied by the surgical robot to the manipulation terminal comprises:

    deriving a commanded angle $\theta$ for the joint in the manipulation terminal from the commanded position and posture information Xd through inverse kinematics;
    calculating a theoretical output torque Fs by using the commanded angle $\theta$ as an input to a dynamic calculation;
    calculating a torque required by the joint in the manipulation terminal for movement from a current position and posture to the commanded position and posture by using the commanded angle $\theta$ as an input to a position and posture controller;
    calculating a torque Fc of the external environmental force from an equivalent torque F, gravity and friction compensation torques N and the theoretical output torque Fs, wherein the equivalent torque F is sensed by a force sensor under an action of the external environmental force; and
    obtaining the drive information through compensating the torque required by the joint in the manipulation terminal for movement from the current position and posture to the commanded position and posture with the torque Fc of the external environmental force.

4.  The control method for the surgical robot of claim 3, wherein the external environmental force comprises a resistance torque Fa of a surgical object to the manipulation terminal and a traction torque f applied by an operator to the manipulation terminal, wherein the equivalent torque F satisfy F= Fs+N+Fa+f and the torque Fc of the external environmental force satisfy Fc= F-Fs-N.

5.  The control method for the surgical robot of claim 3, wherein the calculation performed by the position and posture controller comprises:
    calculating the torque required by the joint in the manipulation terminal for movement from the current position and posture to the commanded position and posture based on the commanded position and posture, the current position and posture, a commanded speed, and a current speed of the joint in the manipulation terminal.

6.  The control method for the surgical robot of claim 5, wherein the commanded speed is obtained by performing a differential calculation on the commanded position and posture.

7. The control method for the surgical robot of claim 1, wherein the first feedback information comprises commanded position and posture information for the joint in the manipulation terminal, wherein the first feedback information comprises an impedance control model of the external environmental force over the joint in the manipulation terminal.

8. The control method for the surgical robot of claim 7,

wherein compensating the drive information applied by the surgical robot to the manipulation terminal comprises:
deriving a commanded angle $\theta$ for the joint in the manipulation terminal from commanded position and posture information Xd through inverse kinematics;
calculating a theoretical output torque Fs by using the commanded angle $\theta$ as an input to a dynamic calculation;
calculating a first torque in Cartesian space using the impedance control model based on a position and posture difference between a current position and posture and a commanded position and posture of the manipulation terminal and a speed difference between a current speed and a commanded speed of the manipulation terminal;
converting the first torque into a second torque that the joint is subject to through a transposition of a Jacobian matrix of the joint at a current angle thereof;
deriving a third torque of the joint through compensating the joint in the manipulation terminal with a corresponding friction feedforward f; and
deriving the drive information from the theoretical output torque Fs, the third torque and the second torque;
or
compensating the drive information applied by the surgical robot to the manipulation terminal comprises:

deriving a commanded angle $\theta$ for the joint in the manipulation terminal from commanded position and posture information Xd through inverse kinematics;
calculating a theoretical output force and a torque Fs thereof by using the commanded angle $\theta$ as an input to a dynamic calculation;
calculating a first force and a first torque thereof in Cartesian space using the impedance control model based on a position and posture difference between a current position and posture and a commanded position and posture of the manipulation terminal and a speed difference between a current speed and a commanded speed of the manipulation terminal;
converting the first force and the first torque thereof into a second force and a second torque thereof that the joint is subject to through a transposition of a Jacobian matrix of the joint at a current angle thereof;
deriving a third force and a third torque of the joint through compensating the joint in the manipulation terminal with a corresponding friction feedforward f; and
deriving the drive information from the theoretical output force and the torque thereof Fs, the third force and the third torques thereof, and the second force and the second torque thereof.

9. The control method for the surgical robot of claim 8, wherein an input to the impedance control model is derived using a process comprising the steps of:

calculating a position and posture variation for the joint through admittance control based on an equivalent torque F output from a force sensor under an action of the external environmental force;
calculating the position and posture difference between the current position and posture and the commanded position and posture of the manipulation terminal based on the position and posture variation through forward kinematics; and
taking the position and posture difference as the input to the impedance control model.

10. The control method for the surgical robot of claim 1, wherein the manipulation terminal comprises a robotic arm and/or a manipulator, wherein the first feedback information comprises commanded position and posture information for a joint in the robotic arm and/or the manipulator, and wherein the manipulator is configured to fix a surgical instrument thereto and guide the surgical instrument to perform a surgical operation.

11. A readable storage medium, storing a program thereon, wherein the program, when executed, implements the control method for a surgical robot as defined in any one of claims 1 to 10.

12. A surgical robot, comprising a manipulation terminal, the manipulation terminal comprising a robotic arm and/or a manipulator for guiding a surgical instrument to perform a surgical operation, wherein the manipulation terminal is controlled using the control method for a surgical robot as defined in any one of claims 1 to 10.

13. A surgical robot system, comprising a control device, a navigation device and a manipulation terminal, the navigation device configured to track a current position and posture of the manipulation terminal and feed the position and posture information back to the control device, the control device configured to control the manipulation terminal using the control method for a surgical robot as defined in any one of claims 1 to 10.

14. The surgical robot system of claim 13, wherein the manipulation terminal comprises a robotic arm and a manipulator for guiding a surgical instrument to perform a surgical operation, the manipulator having a plurality of degrees of freedom, wherein the first feedback information comprises commanded position and posture information for a joint in the robotic arm and/or the manipulator.

Fig. 1

Fig. 2

Fig. 3

Fig. 4

Fig. 5

Fig. 6

Fig. 7

$$\Delta x \rightarrow \boxed{M_d s^2 + B_d s + k_d} \rightarrow \Delta F$$

Fig. 8

$$\Delta F \rightarrow \boxed{\dfrac{1}{M_s s^2 + B_s s + k_s}} \rightarrow \Delta x$$

Fig. 9

## INTERNATIONAL SEARCH REPORT

| | International application No. |
| --- | --- |
| | **PCT/CN2021/128828** |

**A. CLASSIFICATION OF SUBJECT MATTER**

A61B 34/00(2016.01)i

According to International Patent Classification (IPC) or to both national classification and IPC

**B. FIELDS SEARCHED**

Minimum documentation searched (classification system followed by classification symbols)

A61B 34/-

Documentation searched other than minimum documentation to the extent that such documents are included in the fields searched

Electronic data base consulted during the international search (name of data base and, where practicable, search terms used)

CNKI, CNPAT, WPI, EPODOC: 安全, 警戒, 边界, 界线, 范围, 补偿, 抵消, 力, 操作者, 外界, 环境, 区域, 接近, 超出, safety, safe, warning, line, boundary, border, region, force, compensate, reduce, environment, external, approximate, beyond, exceed

**C. DOCUMENTS CONSIDERED TO BE RELEVANT**

| Category* | Citation of document, with indication, where appropriate, of the relevant passages | Relevant to claim No. |
| --- | --- | --- |
| PX | CN 112336461 A (SUZHOU WEICHUANG CHANGXING ROBOTS CO., LTD.) 09 February 2021 (2021-02-09) claims 1-14, description paragraphs [0053]-[0107], figures 1-9 | 1-14 |
| A | US 2014222207 A1 (STRYKER CORPORATION) 07 August 2014 (2014-08-07) description paragraphs [0060], [0098], [0314]-[0321], figure 25 | 1-14 |
| A | US 2014039517 A1 (STRYKER CORPORATION) 06 February 2014 (2014-02-06) entire document | 1-14 |
| A | CN 106965175 A (BEIJING INSTITUTE OF TECHNOLOGY) 21 July 2017 (2017-07-21) entire document | 1-14 |
| A | CN 111870349 A (QIANYUAN YUNLI (BEIJING) ROBOT INTELLIGENT TECHNOLOGY CO., LTD.) 03 November 2020 (2020-11-03) entire document | 1-14 |
| A | CN 110114031 A (ORTHOSOFT INC.) 09 August 2019 (2019-08-09) entire document | 1-14 |

☐ Further documents are listed in the continuation of Box C.   ☑ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **11 January 2022** | **26 January 2022** |

| Name and mailing address of the ISA/CN | Authorized officer |
| --- | --- |
| **China National Intellectual Property Administration (ISA/CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

# EP 4 241 715 A1

**INTERNATIONAL SEARCH REPORT**
Information on patent family members

International application No.

**PCT/CN2021/128828**

| Patent document cited in search report | | | Publication date (day/month/year) | Patent family member(s) | | | Publication date (day/month/year) |
|---|---|---|---|---|---|---|---|
| CN | 112336461 | A | 09 February 2021 | None | | | |
| US | 2014222207 | A1 | 07 August 2014 | US | 2017128147 | A1 | 11 May 2017 |
| | | | | US | 10420619 | B2 | 24 September 2019 |
| | | | | US | 2016089211 | A1 | 31 March 2016 |
| | | | | US | 9795445 | B2 | 24 October 2017 |
| | | | | US | 2015265358 | A1 | 24 September 2015 |
| | | | | US | 9566122 | B2 | 14 February 2017 |
| | | | | US | 2017245955 | A1 | 31 August 2017 |
| | | | | US | 10426560 | B2 | 01 October 2019 |
| | | | | US | 2020022768 | A1 | 23 January 2020 |
| | | | | US | 11179210 | B2 | 23 November 2021 |
| | | | | US | 9226796 | B2 | 05 January 2016 |
| | | | | US | 2015289941 | A1 | 15 October 2015 |
| | | | | US | 9681920 | B2 | 20 June 2017 |
| US | 2014039517 | A1 | 06 February 2014 | US | 2019192249 | A1 | 27 June 2019 |
| | | | | US | 10463440 | B2 | 05 November 2019 |
| | | | | EP | 2879608 | A2 | 10 June 2015 |
| | | | | EP | 2879608 | B1 | 18 March 2020 |
| | | | | CA | 2879414 | A1 | 06 February 2014 |
| | | | | US | 2017000577 | A1 | 05 January 2017 |
| | | | | US | 10350017 | B2 | 16 July 2019 |
| | | | | AU | 2013296278 | A1 | 12 February 2015 |
| | | | | AU | 2013296278 | B2 | 14 June 2018 |
| | | | | CN | 107198567 | A | 26 September 2017 |
| | | | | AU | 2018220167 | A1 | 13 September 2018 |
| | | | | AU | 2018220167 | B2 | 12 September 2019 |
| | | | | CN | 112932672 | A | 11 June 2021 |
| | | | | KR | 20150039801 | A | 13 April 2015 |
| | | | | KR | 102235965 | B1 | 06 April 2021 |
| | | | | US | 2017172680 | A1 | 22 June 2017 |
| | | | | US | 10314661 | B2 | 11 June 2019 |
| | | | | CN | 104736092 | A | 24 June 2015 |
| | | | | KR | 20210118467 | A | 30 September 2021 |
| | | | | US | 2015366629 | A1 | 24 December 2015 |
| | | | | US | 9566125 | B2 | 14 February 2017 |
| | | | | US | 2020030046 | A1 | 30 January 2020 |
| | | | | EP | 3620121 | A1 | 11 March 2020 |
| | | | | AU | 2019275554 | A1 | 02 January 2020 |
| | | | | AU | 2019275554 | B2 | 26 November 2020 |
| | | | | US | 9480534 | B2 | 01 November 2016 |
| | | | | WO | 2014022786 | A2 | 06 February 2014 |
| | | | | AU | 2021201169 | A1 | 11 March 2021 |
| | | | | KR | 20210035922 | A | 01 April 2021 |
| | | | | KR | 102304096 | B1 | 24 September 2021 |
| CN | 106965175 | A | 21 July 2017 | None | | | |
| CN | 111870349 | A | 03 November 2020 | None | | | |
| CN | 110114031 | A | 09 August 2019 | AU | 2019268154 | A1 | 12 December 2019 |
| | | | | AU | 2019268154 | B2 | 11 March 2021 |
| | | | | CA | 3042097 | A1 | 03 May 2018 |

Form PCT/ISA/210 (patent family annex) (January 2015)

17

**INTERNATIONAL SEARCH REPORT**
Information on patent family members

International application No.

**PCT/CN2021/128828**

| Patent document cited in search report | Publication date (day/month/year) | Patent family member(s) | | Publication date (day/month/year) |
|---|---|---|---|---|
| | | EP 3531951 | A1 | 04 September 2019 |
| | | WO 2018076114 | A1 | 03 May 2018 |
| | | AU 2017348662 | A1 | 23 May 2019 |
| | | AU 2017348662 | B2 | 29 August 2019 |
| | | US 2018116739 | A1 | 03 May 2018 |
| | | US 11154372 | B2 | 26 October 2021 |
| | | US 2018116740 | A1 | 03 May 2018 |
| | | US 11179207 | B2 | 23 November 2021 |

Form PCT/ISA/210 (patent family annex) (January 2015)